# EUROPEAN PATENT APPLICATION

(11) **EP 1 057 459 A1**
(43) Date of publication of application: **06.12.2000**
(21) Application number: 99420223.2
(22) Date of filing: 09.11.1999
(51) Int. Cl.: A61F 2/06

(54) **Radially expandable stent**

(30) Priority: 01.06.1999 US 137009; 20.09.1999 US 399543
(71) Applicant: NUMED, INC., Nicholville, NY 12965 (US)
(72) Inventor: Tower, Allen J., North Lawrence, NY 12965 (US)
(74) Representative: Vuillermoz, Bruno

(57) **Abstract**

A radially expandable stent for intravascular implantation has a tubular structure defined by an open-ended interior adapted to receive an inflatable portion of a balloon catheter for percutaneous delivery into a patient lumen. The stent is formed from a plurality of malleable wire sections, each section having a series of sinusoidal bends with adjacent wires being welded to one another at the apex of each of said sinusoidal bends. The wire sections are formed circumferentially to define a unitary tubular structure. Preferably, a layer of a fluid impermeable material can be disposed over the wire-mesh frame of the stent, the material being synthetic or biological in nature, prior to implantation.

## Description

### FIELD OF THE INVENTION

This invention relates to the field of medical implantable devices, and more particularly to an improved intravascular stent which can be percutaneously implanted into a patient body lumen and radially expanded, such as by balloon catheterization.

### BACKGROUND OF THE INVENTION

The basic concepts of intravascular stents has been known for a number of years. Various types of stents have been proposed and patented, including self-expanding spring types, compressed spring types, mechanically actuated expandable devices, and the like. More recently, expandable sleeves have been proposed such as those described in Palmaz, U.S. Patent No. 4,733,665.

In the aforementioned '665 patent, a stainless steel or other metallic sleeve includes a plurality of slots forming a permeable mesh. The sleeve is positioned transluminally, and then expanded by a balloon catheter through the elastic limit of the metal so as to permanently deform the sleeve into supporting contact with the interior surface of a blood vessel.

Stents, such as those described by Palmaz, are designed to be expanded a single time and only to a specified diameter. Therefore, even properly implanted stents of this type may not produce a satisfactory result, particularly in procedures involving infants and young children, each having blood vessels which will naturally increase in size over time. Moreover, the radial expansion of such stents also produces a corresponding significant lateral or axial shrinkage.

More recently, a number of implantable stents have subsequently been proposed and patented which are made from a highly malleable material which allows the stent to be locally deformed. Such stents are described, for example, in Applicant's patents, U.S. Patent Nos. 5,868,783 and 5,389,106, among others. The above stents are manufactured from fine wires made from a material which has as much spring memory removed as possible, thereby readily allowing plastic deformation. A key advantage of these types of stents are that they can easily be crimped onto a balloon catheter for transluminal delivery and then radially expanded upon inflation of an implanted balloon. Moreover, these types of stents can later be reexpanded to a larger diameter after implantation, which is especially useful for patients who are children and without requiring the involvement or rigor of invasive open heart surgery. Because the stents are locally deformable, the stent conforms generally to the shape of the balloon, allowing inflatable portions having separate differently shaped portions to be used for different types of procedures, such as described in U.S. Patent No. 5,695,498, without significant risk that the stent will be dislodged from the balloon in the event of misalignment.

Though it is desired to maintain the highly flexible nature of the above malleable stents, there is also a competing need to provide uniformity in the expansion characteristics thereof. Moreover, there is a similar need to reduce the amount of axial shrinkage attributable to radial expansion of the stent.

### SUMMARY OF THE INVENTION

It is a primary object of the present invention to improve the state of the art of medical implantable devices.

It is a further primary object to provide an improved intravascular stent which can be implanted using a balloon catheter or other noninvasive device, the stent being suitable for several different procedures including those for infants and young children having blood vessels which will grow over time.

It is another primary object of the present invention to provide a radially expandable stent which can be expanded without significant axial shrinkage.

Therefore and according to a preferred aspect of the invention, there is provided a radially expandable stent for intravascular implantation, said stent having a tubular structure defined by an open-ended interior which is sized to allow the stent to be placed in overlaying fashion onto an inflatable portion of a balloon catheter, said stent comprising:
a plurality of cylindrical fine wire ribbons arranged in an axial configuration and interconnected to form a unitary tubular structure, each fine wire ribbon having a periodic series of substantially sinusoidal shaped bends formed along the entire circumferential length thereof, wherein each of said shaped bends includes an apex which is welded to an apex of a corresponding sinusoidal shaped bend of an adjacent wire ribbon.

Preferably, each fine wire ribbon or section is made from a soft highly malleable material, such as an annealed platinum (Pt), Au-Ni, or platinum-iridium, which has as much of its spring memory removed as possible to permit local plastic deformation.

According to a preferred forming method, the substantially sinusoidal shaped bends are initially formed by taking individual fine wires and winding them about a pair of spaced rows of pins, thereby forming an alternating series of bends. When completed, each apex of each sinusoidally shaped bend of a formed wire section is welded to a corresponding apex or elbow of an adjacently disposed and similarly formed wire section. Finally, the interconnected wire sections are wound around a cylindrical mandrel to form an overall tubular structure with the free ends of each fine wire section also being welded together. Each of the shaped bends are formed along the longitudinal axis of the stent, allowing a conveniently low surface profile.

In operation, the stent can be conveniently crimped or otherwise placed with the low profile onto an expandable portion of a balloon catheter and delivered percutaneously into a patient lumen of interest. Inflation of the expandable portion of the balloon catheter causes the stent to radially expand into supporting contact with the interior of a lumen wall. The balloon catheter can subsequently be deflated and removed from the lumen, leaving the stent in place. Each apex is rounded, meaning there are no sharp edges at either end of the stent to puncture the catheter or the blood vessel into which the stent is implanted.

Moreover, the above stent can include a coated layer which can easily be applied, such as by dipping the stent into a flexible biocompatible material or by placement off a flexible distensible membrane onto the wire mesh frame. The dipping process can occur either prior to or after the final forming step. Alternately, a biological material can also be sutured or otherwise attached to the stent to serve as a fluid impermeable barrier.

An advantage of the present invention is that the above-described stent can later be expanded to a larger diameter as needed after initial implantation without requiring open heart surgery. The stent can be reexpanded by reinserting a balloon catheter, aligning the inflatable portion of the catheter with the implanted stent, and additionally radially expanding the stent as needed. Due to its malleable nature, the stent can be used, for example, in conjunction with a dual balloon catheter which allows the stent to be initially secured to the inflatable portion by inflating a first balloon to a first inflation pressure and then subsequently inflating a second overlaying balloon to a second greater inflation pressure which radially expands the attached stent to a predetermined size, without sharp edges or ends.

The ability to reexpand the implanted stent is especially advantageous for patients such as infants and small children who have blood vessels which are subject to growth changes. Furthermore use of a radioopaque material, such as platinum or platinum-iridium wire, is also useful in tracking the location of the stent for implantation and subsequent expansion procedures.

Another advantage provided by the above-described stent is that substantial axial shrinkage of the stent is avoided during radial expansion. However, the welded interconnection between the adjacent cylindrical fine wire sections allows greater uniformity in the radial expansion of the stent while yet still permitting local deformation to those sections acted upon by a radial force.

The described configuration also provides a stent having good flexibility, dimensional stability, and immunity to fatigue and corrosion.

These and other objects, features and advantages will become readily apparent from the following Detailed Description which should be read in conjunction with the accompanying drawings.

### BRIEF DESCRIPTION OF THE DRAWINGS

Fig. 1 is a top perspective view of an intravascular stent made in accordance with the prior art;
Fig. 2 is a side view of the prior art stent of Fig. 1, showing the axial shrinkage thereof after the stent is expanded;
Fig. 3 is a top view of a planar sheet made up of a plurality of interconnected fine wire sections representative of a radially expandable stent made in accordance with a preferred embodiment of the present invention prior to final assembly of the stent;
Fig. 4 is a side view of the completed stent of Fig. 3;
Fig. 5(a) is a side elevational view, partially in section, of the welded stent of Fig. 4 when attached to an uninflated balloon catheter in an occluded blood vessel;
Fig. 5(b) is the side elevational view of the stent of Figs. 4 and 5(a), partially in section, of the stent of Fig. shown after expansion and removal of the catheter; and
Fig. 6 is an end view of the stent of Figs. 4-5(b) having an impermeable biological membrane attached thereto.

### DETAILED DESCRIPTION

The following discussion pertains to an radially expandable intravascular stent made in accordance with a preferred embodiment of the present invention. Throughout the course of discussion, terms such as "top" , "side", "lateral", and the like are used. These terms are used to provide a frame of reference with regard to the accompanying drawings and are not intended to be limiting of the present invention as claimed.

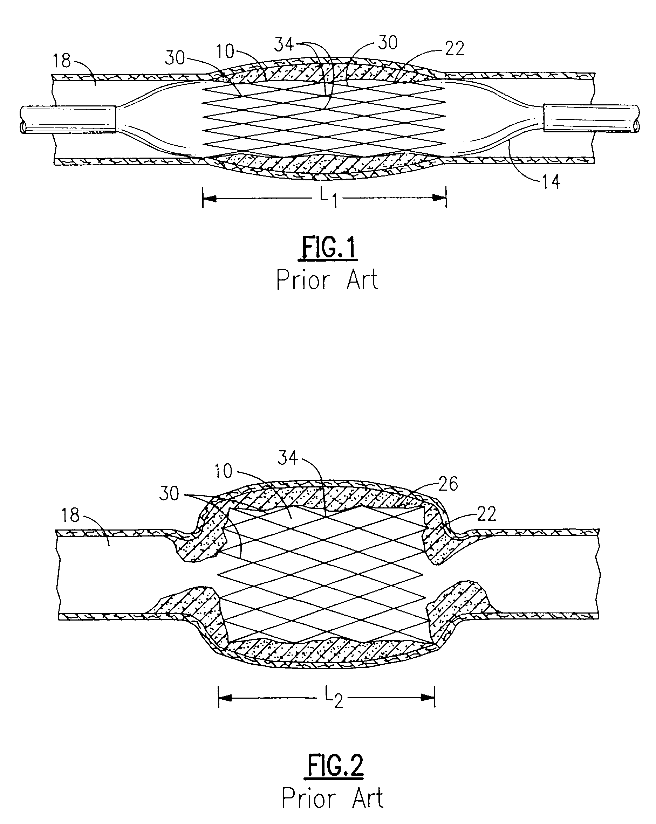

To provide sufficient background, Figs. 1 and 2 illustrate a radially expandable prior art stent 10 that is described in the afore-mentioned U.S. Patent No. 4,733,665, issued to Palmaz et al. In brief, the stent 10 is made from a sleeve-like member made from stainless steel, tantalum or other metal material. The sleeve is formed of a series of interconnected criss-crossing elongate members 30 which are welded throughout at intersecting points 34 to define a unitary cylindrical structure. As shown in Fig. 1, and its initial formed state, the stent 10 defined by a first radial diameter, is placed over a collapsed balloon catheter 14 and positioned within a blood vessel 18 of interest having an occluded portion 22.

The collapsed catheter balloon 14 is then inflated to allow the stent 10 to controllably assume a second radial diameter, shown in Fig. 2, in contact with the interior wall 26 of the blood vessel 18. The stent 10 when acted upon by the balloon 14 is caused to uniformly expand. As is apparent, an increase in radial size of the stent 10 causes a corresponding decrease in axial length, shown figuratively as the difference between L₁ and L₂, whereby the described stent 10 could lose as much as 40-50 percent or more in length due to axial shrinkage.

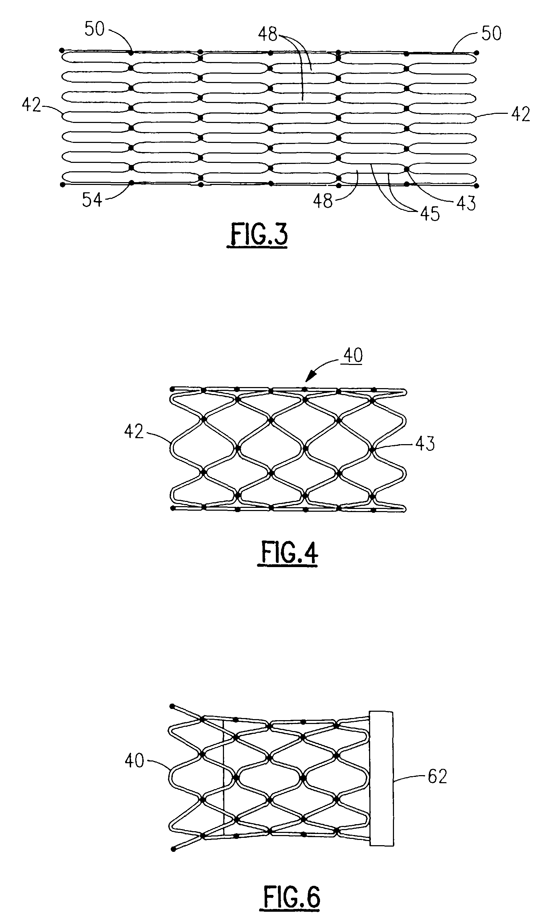

Referring now to Figs. 3-5(b), an improved intravascular stent 40 made in accordance with the present invention is herein described. According to this specific embodiment, the stent 40 is made from a plurality of fine wire sections 42. The wires each are made from a highly malleable material which has been fully annealed to remove as much spring memory as possible. According to the specific embodiment, the wire material is a composition of approximately 90 % platinum and 10% iridium. Other suitable materials include pure platinum, (Au-Ni), gold alloys, and literally any malleable and bio-implantable material.

As shown in the partial assembly view of Fig. 3, each individual fine wire section 42 is formed with a series of alternating substantially sinusoidally shaped bends 48. Preferably, each of the bends 48 are formed by tightly winding a fine wire between a plurality of spaced rows of projecting cylindrical pins of an assembly fixture (not shown) along a serpentine winding path. When completed, each resulting wire section 42 includes a predetermined number of substantially sinusoidal shaped bends 48 and a pair of free ends 50, 54 at opposing ends thereof. Still referring to Fig. 3, each individual substantially sinusoidal bend 48 includes a pair of sides 45, one of which is shared with an adjacent bend, which commonly converge into a common apex 43. As noted, the bends 48 alternate and therefore each apex 43 is disposed on either lateral side of the wire section 52. According to this embodiment, each of the fine wires has a diameter of about 0.013 inches, though it should be readily apparent that this parameter can be easily be varied (.009 inches, .011 inches, etc.), depending on the application. A predetermined number of bends 48 are formed based on the number of rows of pins provided on the assembly fixture (not shown). According to the present embodiment, eight (8) bends are formed in each wire section 42.

Still referring to Fig.3, adjacent wire sections 42 are similarly formed and disposed on the assembly fixture (not shown) such that a predetermined number of wire sections are adjacently positioned in a planar arrangement. The adjacent wire sections 42 are interconnected to one another by welding the apices 43 of adjacent wires together.

The resulting planar wire mesh sheet can then removed from the assembly fixture (not shown) and be rolled onto a cylindrical mandrel (not shown) having a predetermined diameter with the free ends 50, 54 of each individual wire section 52 being welded together in a circumferential fashion to form a closed structure with a circular cross section. According to the present embodiment, a mandrel (not shown) having a diameter of 0.138 inches is used, but as noted above, this parameter can easily be varied, depending on the intended application.

The overall axial length of the stent 40 is dictated by the number of interconnected wire sections 42. In the present embodiment, seven (7) coupled wire sections 42 are used to define a structure having an axial length of approximately 1.5 inches. The apices 43 of the outermost or end wire sections 42 are free standing and form the outer edges of the finished stent, as shown in Fig. 4.

Optionally and depending on the particular use of the stent, a distensible coating layer can be added to the wire mesh frame of the formed stent 40, such as by dipping the stent 40 into a receptacle (not shown) having a melted synthetic polymer which forms a distensible membrane when treated. A suitable membrane is described, for example, in U.S. Patent No. 5,389,106.

According to the present embodiment, the planar sheet can be dipped into a container of the melted polymer (not shown) prior to winding the sheet of Fig. 3 about the cylindrical mandrel or the stent can be dipped after wrapping the stent about the mandrel. Alternately, a section of a flexible polymer or polyamide balloon can be secured to the wire mesh of the stent to form a fluid impermeable membrane.

As depicted in Fig. 6, a tubular section 62 of a biological material can be attached to the wire frame of the stent 40 in lieu of a synthetic material. Certain biological materials are elastic by nature and can be easily assembled to the stent 40. For example, and as described in concurrently filed and commonly assigned USSN 60/137,008, a tubular portion of a bovine jugular vein can be sutured or otherwise attached to the interior wall of a stent 40 as a replacement valve assembly. Similar tubular portions without internal valve leaflets can similarly be attached as a fluid impermeable membrane.

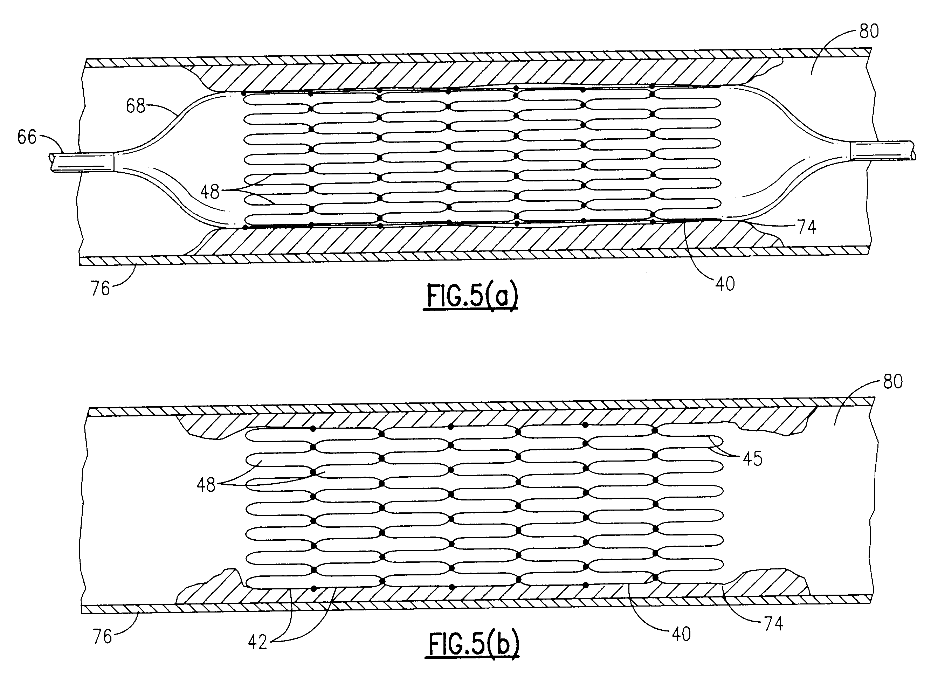

Referring to Figs. 5(a) and 5(b), the above-described stent 40 is placed onto the inflatable (balloon) portion 68 of a balloon catheter 66 and can be crimped or otherwise placed into intimate contact therewith. The balloon catheter 66 and stent 40 are then percutaneously implanted using a guide wire (not shown) in a manner which is commonly known into a patient body lumen. Upon proper placement of the above assembly, the inflatable portion 68 is inflated by an appropriate inflation pressure to a predetermined diameter, thereby expanding the stent 40 radially until the stent contacts the interior 74 and more particularly an occluded portion 78 of a blood vessel 80. Preferably, and by making the stent from a radioopaque material, the position of the stent 40 can easily be tracked without requiring image bands, such as those typically added to the ends of the inflatable portion 68 of the catheter 66.

As the inflatable portion of the balloon catheter 66 expands, each of the sinusoidal bends 48 of the stent 40 are caused to open; that is, the spacing between each of the sides 45 is increased. Upon contacting and supporting the interior 74 of the vessel 80, the inflatable portion 68 can then be deflated and removed from the patient's body while the stent 40 remains in supporting contact with the occluded portion 78.

The welded interconnection of the wire sections 42 provides additional uniformity in the overall expansion characteristics of the stent 40. However, as shown in comparison of Figs. 5(a) and 5(b), the radial expansion of the stent 40 does not produce significant axial shrinkage.

An advantage of the described stent is that upon growth of the lumen, such as rapid growth demonstrated in infants and young children, the stent can be further enlarged by reimplanting the balloon within the lumen and reinflating the balloon into contact with the stent, allowing the stent to be further expanded without invasive procedures, such as removal and reimplantation of a new larger stent, being required.

Though the invention has been described with regard to a preferred embodiment, it will be readily apparent that other variations and modifications can easily be imagined by those of adequate skill in the field which are within the spirit and scope of the invention as presently claimed.

## Claims

1. A radially expandable stent for intravascular implantation, said stent being defined by a cylindrical tubular structure having an open-ended interior adapted to receive an inflatable portion of a balloon catheter for percutaneous delivery into a patient lumen, said stent being characterized by:
a plurality of circumferential fine wire ribbons axially interconnected to form a unitary tubular member, each wire ribbon including a periodic series of substantially sinusoidal shaped bends along the entire length thereof, wherein each of said shaped bends including an apex which is welded to an apex of an adjacent wire ribbon.

2. The stent of Claim 1, further characterized in that each of said wires is made from a highly malleable material which permits local deformation.

3. The stent of Claim 1, further characterized in that a nonpermeable, coated layer over substantially the entire axial length thereof.

4. The stent of Claim 3, further characterized in that said coated layer is made from a synthetic non-latex, non-vinyl polymer.

5. The stent of Claim 3, further characterized in that said coated layer is a polyamide material.

6. The stent of Claim 3, further characterized in that said nonpermeable coated layer is made from a biocompatible material.

7. The stent of Claim 3, further characterized in that said nonpermeable coated layer includes a tubular section of a biological material which is sutured to the stent.

8. The stent of Claim 2, further characterized in that each of said circumferential wire ribbons are made from a malleable material having substantially all of its spring memory removed.

9. The stent of Claim 8, further characterized in that said malleable wires are made from an annealed alloy comprising a combination of platinum and iridium.

10. An apparatus for intravascular implantation into a body lumen, said apparatus comprising:
a balloon catheter having an inflatable portion for delivering said stent to an intravascular implantation site and for radially expanding said stent for implantation into said vessel,
a radially expandable stent having a tubular structure, said apparatus being
characterized in that said tubular structure is defined by an open ended interior and includes a plurality of circumferential fine wire segments, each wire segment having a formed series of substantially sinusoidal bends, each of said bends having a pair of spaced sides converging into an apex, said wire segments being axially interconnected by welding apices of adjacent circumferential wire sections together and in which each of said wire segments of said stent being made from a highly malleable material which permits local plastic deformation thereof.

11. The apparatus of Claim 10, further characterized in that a distensible membrane interconnecting portions of said tubular structure to form an fluid-impermeable wall surface disposed between first and second ends of said stent about a longitudinal axis.

12. The apparatus of Claim 11, further characterized in that said distensible membrane comprises a synthetic non-latex, non-vinyl polymer.

13. The apparatus of Claim 10, further characterized in that said distensible membrane comprises a tubular section of a biological material which is sutured to said stent.

14. The apparatus of Claim 10, further characterized in that said distensible membrane substantially extends over the axial length of said stent.

15. The apparatus of Claim 10, further characterized in that said malleable wire segments are made from a soft material which has been annealed to remove as much spring memory as possible.

16. The apparatus of Claim 15, further characterized in that said stent material is platinum.

17. The apparatus of Claim 15, further characterized in that said stent material is niconel.

18. The apparatus of Claim 15, further characterized in that said stent material is an alloy consisting of platinum and iridium.

19. The apparatus of Claim 18, further characterized in that said alloy includes about 90 % platinum and 10% iridium.

20. A radially expandable stent for intravascular implantation, said stent having a tubular structure defined by opposing first and second ends and a longitudinal axis, and characterized by:
a plurality of wire sections, each wire section being made form a highly malleable material having a series of substantially sinusoidal bends formed over an circumferential length, each of said bends having an apex in which adjoining bends of adjacent circumferential wire sections are welded together to hold said tubular structure in a substantially cylindrical configuration.

21. The stent of Claim 20, further characterized in that a distensible membrane interconnecting portions of said tubular structure, said membrane being made from a fluid impermeable material disposed between said first and second ends about said longitudinal axis.

22. The stent of Claim 21, further characterized in that said distensible membrane comprises a synthetic non-latex, non-vinyl polymer.

23. The stent of Claim 21, further characterized in that said distensible membrane substantially extends from said first end to said second end of said stent.

24. The stent of Claim 20, further characterized in that said tubular structure includes an open ended interior adapted to receive an inflatable balloon portion of a balloon catheter for intravascular placement and radial expansion therewith.

25. The stent of Claim 20, further characterized in that each malleable wire section comprises a fine platinum-iridium wire which is annealed to remove substantially all shape memory therefrom.

26. A method for manufacturing an intravascular stent, said method comprising the step of:
forming a series of repeatable bends into a fine wire ribbon made from a highly malleable material, said method being characterized by the additional steps of:
connecting adjacent wire ribbons together into by welding adjoining bends together in an axial configuration to form a wire mesh frame; and
connecting free ends of each of said wire ribbons to form a tubular structure having a plurality of circumferential wire ribbons.

27. The method of Claim 26, further characterized by the step of applying a fluid impermeable layer to said wire mesh frame.

28. The method of Claim 27, wherein said layer-applying step is further characterized by the step of suturing a tubular biological section to said stent following said connecting step.

29. The method of Claim 27, wherein said layer-applying step is further characterized by the step of dipping said stent into a container of a fluid impermeable material.
